# FASCICULE DE BREVET EUROPEEN

(11) **EP 4 031 855 B1**
(45) Date de publication et mention de la délivrance du brevet: **24.12.2025**
(21) Numéro de dépôt: 20774938.3
(22) Date de dépôt: 16.09.2020
(51) Int. Cl.: G01N 21/84, G01N 33/487

(54) **MÉTHODE ET LECTEUR PORTABLE D'ANALYSE D'ÉCHANTILLON DE LIQUIDE BIOLOGIQUE**
VERFAHREN UND TRAGBARER LESER ZUM ANALYSIEREN EINER BIOLOGISCHEN PROBE
METHOD AND PORTABLE READER FOR ANALYSING A BIOLOGICAL FLUID SPECIMEN

(30) Priorité: 17.09.2019 FR 1910244
(43) Date de publication de la demande: 27.07.2022
(73) Titulaire: Iki, 31106 Toulouse Cedex 1 (FR)
(72) Inventeur: JULIEN, Guillaume, 31600 Muret (FR); LEBLANC, Morgane, 31000 Toulouse (FR); BOUISSOU, Bernard, 31270 Cugnaux (FR); FABBRO, Didier, 31390 Carbonne (FR); CALVE, Julien, 31830 Plaisance du Touch (FR); LUMBIERRES, Philippe, 31390 Carbonne (FR); CAUCHOIS, Cyril, 31220 Saint Julien sur Garonne (FR); CAU, Jean-Christophe, 31400 Toulouse (FR)
(74) Mandataire: Santarelli
(86) Numéro de dépôt international: PCT/EP2020/075811
(87) Numéro de publication internationale: WO 2021/052983

(56) Documents cités:
- EP-A1- 2 975 408
- EP-A1- 3 499 220
- WO-A1-97/26083
- US-A- 5 281 395
- US-A- 5 611 995
- US-A1- 2012 300 211
- US-A1- 2016 103 075
- US-A1- 2016 370 366
- US-B1- 6 867 051

## Description

### Domaine technique de l'invention

La présente invention concerne un lecteur portable d'analyse d'échantillon de liquide biologique et une méthode d'analyse d'échantillon de liquide biologique. Elle s'applique, en particulier, à un système d'analyse d'échantillon de liquide biologique comportant une cartouche de prélèvement d'échantillon de liquide biologique insérée dans le lecteur portable.

### Technique antérieure

Il est courant, chez les professionnels de la santé, de pouvoir faire analyser un échantillon de liquide biologique telle que de l'urine ou encore de la salive. Il est connu d'utiliser des bandelettes équipées de quelques éléments réactifs dont la couleur peut varier au contact du liquide biologique après un trempage de la bandelette dans le liquide biologique. L'interprétation du changement de couleurs peut être effectuée soit à l'œil nu selon un tableau d'analyse permettant de corréler les couleurs à une concentration de composée dans le liquide biologique, soit par introduction de la bandelette dans un appareil de lecture encombrant permettant l'analyse de la bandelette.

Il a déjà été tenté de résoudre ce problème en proposant au grand public de tremper lui-même des bandelettes équipées d'éléments réactifs dans un bocal comprenant par exemple de l'urine, et d'analyser lui-même à l'œil nu, le changement de couleur des réactifs et d'interpréter les couleurs selon une notice fournie avec la bandelette.

Cette solution à domicile ne renseigne pas toujours de manière fiable l'utilisateur, l'utilisateur pouvant avoir du mal à discerner un changement de couleur au regard de la notice, le temps de trempage de la bandelette dans le liquide biologique n'étant pas forcément maitrisé par l'utilisateur, le temps de latence entre la fin de trempage et l'interprétation des couleurs des réactifs n'étant également pas toujours maitrisé par l'utilisateur.

Les documents US 2012/300211, US 2016/370366 et US 5 661 995 divulguent des exemples de systèmes d'analyse d'échantillon de liquide biologique.

### Présentation de l'invention

La présente invention vise à remédier à ces inconvénients avec une approche totalement novatrice.

Selon un premier aspect, la présente invention se rapporte à un système d'analyse d'échantillon de liquide biologique tel que défini dans la revendication 1. Des modes de réalisation préférés sont définis par les revendications dépendantes.

Selon un deuxième aspect, la présente invention se rapporte à une méthode d'analyse d'échantillon de liquide biologique, la méthode étant mise en œuvre par le système d'analyse d'échantillon de liquide biologique. La méthode est telle que définie dans la revendication 4. Des modes de mises en œuvre préférés sont définis par les revendications dépendantes.

### Brève description des figures

D'autres avantages, buts et caractéristiques de la présente invention ressortent de la description qui suit faite, dans un but explicatif et nullement limitatif, en regard des dessins annexés, dans lesquels :
[Fig. 1] la figure 1 est une vue schématique en perspective d'un premier mode de réalisation d'un système d'analyse d'échantillon liquide biologique comportant une cartouche de prélèvement d'échantillon liquide biologique et un lecteur d'analyse de l'échantillon du liquide biologique.
[Fig. 2] la figure 2 est une vue schématique éclatée en perspective de dessus du lecteur d'analyse comportant la cartouche de prélèvement d'échantillon liquide biologique selon le premier mode de réalisation.
[Fig. 3] la figure 3 est une vue schématique éclatée en perspective de dessus de la cartouche de prélèvement d'échantillon liquide biologique selon le premier mode de réalisation.
[Fig. 4] la figure 4 est une vue schématique en perspective d'un second mode de réalisation d'un système d'analyse d'échantillon liquide biologique comportant une cartouche de prélèvement d'échantillon liquide biologique et un lecteur d'analyse de l'échantillon du liquide biologique.
[Fig. 5] la figure 5 est une vue schématique éclatée en perspective de dessus du lecteur d'analyse comportant la cartouche de prélèvement d'échantillon liquide biologique selon le second mode de réalisation.
[Fig. 6] la figure 6 est une vue schématique éclatée en perspective de dessus de la cartouche de prélèvement d'échantillon liquide biologique selon le second mode de réalisation.
[Fig.7] la figure 7 est une vue schématique en coupe longitudinale du lecteur d'analyse comportant la cartouche de prélèvement d'échantillon liquide biologique selon le second mode de réalisation de l'invention et selon un premier positionnement de la cartouche de prélèvement.
[Fig.8] la figure 8 est une vue schématique en coupe longitudinale du lecteur d'analyse comportant la cartouche de prélèvement d'échantillon liquide biologique selon le second mode de réalisation de l'invention et selon un second positionnement de la cartouche de prélèvement.
[Fig. 9] la figure 9 est une vue schématique en perspective d'un troisième mode de réalisation d'un système d'analyse d'échantillon liquide biologique comportant une cartouche de prélèvement d'échantillon liquide biologique et un lecteur d'analyse de l'échantillon du liquide biologique.
[Fig. 10] la figure 10 est une vue schématique éclatée en perspective de dessus du lecteur d'analyse comportant la cartouche de prélèvement d'échantillon liquide biologique selon le troisième mode de réalisation.
[Fig. 11] la figure 11 est une vue schématique éclatée en perspective de dessous du lecteur d'analyse comportant la cartouche de prélèvement d'échantillon liquide biologique selon le troisième mode de réalisation.
[Fig.12] la figure 12 est une vue schématique en coupe longitudinale du lecteur d'analyse comportant la cartouche de prélèvement d'échantillon liquide biologique selon le troisième mode de réalisation de l'invention et selon un premier positionnement de la cartouche de prélèvement.
[Fig.13] la figure 13 est une vue schématique en coupe longitudinale du lecteur d'analyse comportant la cartouche de prélèvement d'échantillon liquide biologique selon le troisième mode de réalisation de l'invention et selon un second positionnement de la cartouche de prélèvement.
[Fig.14] la figure 14 est un organigramme d'une méthode d'analyse d'échantillon de liquide biologique mise en œuvre par le système d'analyse d'échantillon de liquide biologique des modes de réalisation des figures précédentes.

### Description des modes de réalisation

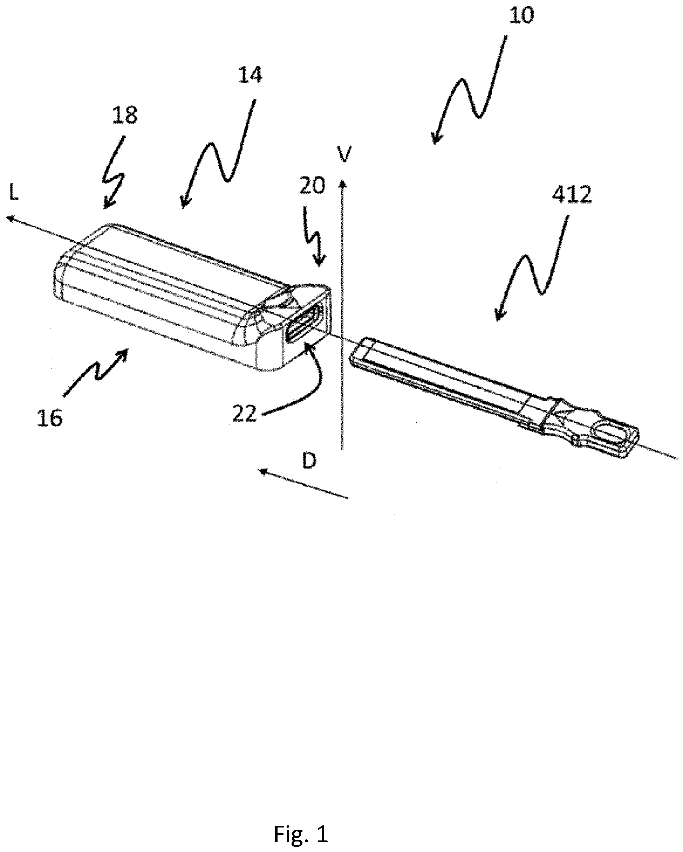

Selon la figure 1, un système 10 d'analyse d'échantillon liquide biologique comprend un dispositif, ou cartouche 412 de prélèvement d'échantillon liquide biologique et un lecteur 14 portable d'analyse d'échantillon de liquide biologique. À des fins de positionnement des éléments des uns avec les autres, et de façon non limitative au regard de l'orientation globale de la cartouche 412 de prélèvement et du lecteur 14, des orientations gauche et droite sont définies selon un axe longitudinal L et des orientations dessus et dessous, ou supérieure et inférieure, sont définies selon un axe vertical V. La cartouche 412 de prélèvement d'échantillon liquide biologique est destinée à être insérée dans le lecteur 14 portable d'analyse de liquide biologique. Le lecteur 14 comprend un boîtier 16 de lecteur 14 s'étendant globalement selon l'axe longitudinal L entre deux extrémités 18, 20 latérales gauche et droite. Le boîtier 16 de lecteur 14 comprend une ouverture latérale d'accès 22 destinée à une insertion selon une direction D longitudinale ou une extraction de la cartouche 412 de prélèvement.

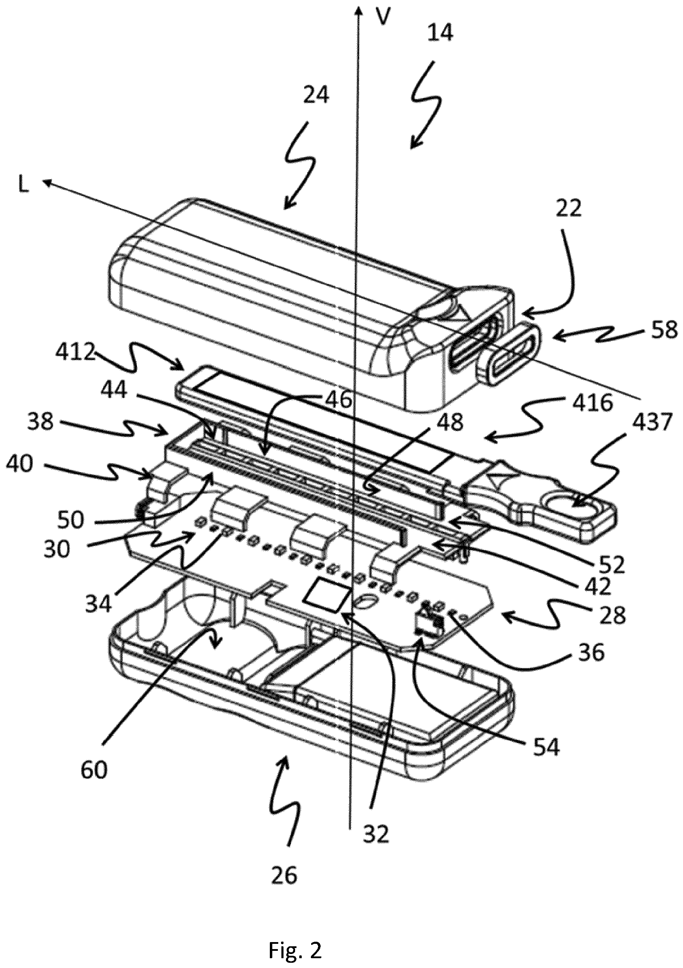

Selon la figure 2 et selon un premier mode de réalisation ne relevant pas de l'invention, le lecteur 14 de la figure 1 comprend le boîtier 16 de lecteur 14 comprenant un fond de boîtier 26 de lecteur 14 et un couvercle 24 de lecteur 14 destiné à fermer le boîtier 16 de lecteur 14. Le lecteur 14 comporte également une carte électronique 28 à circuit imprimé agencée en fond de boîtier 26 de lecteur 14. La face supérieure de la carte électronique 28 à circuit imprimé comporte une pluralité d'ensembles 30 émetteur-récepteur optique, les ensembles 30 émetteur-récepteur optique de la pluralité d'ensembles 30 étant agencés les uns à la suite des autres selon l'axe longitudinale L.

Le lecteur 14 comporte une unité électronique de contrôle 32, avantageusement agencée sur la carte électronique 28 à circuit imprimé, et reliée électriquement à la pluralité d'ensembles 30 émetteur-récepteur optique. L'unité électronique de contrôle 32 est configurée pour commander chaque ensemble 30 émetteur-récepteur optique dans un mode d'émission de lumière et de réception de la lumière réfléchie de sorte à pouvoir effectuer une lecture optique à des fins d'analyse de liquide biologique.

Plus particulièrement chaque ensemble 30 émetteur-récepteur optique comporte une source de lumière 34, de préférence blanche et un capteur optique 36, de préférence type capteur d'image, de sorte que l'unité électronique de contrôle 32 puisse effectuer une analyse de couleur d'éléments réactifs à changement de couleur au contact de liquide biologique.

A titre d'exemple non limitatif, le capteur d'image peut être un capteur de type capteur rouge, vert et bleu, de sorte que l'information de couleur communiquée à l'unité électronique de contrôle par le capteur est une information relative au ratio de chaque couleur primaire détecté par le capteur. Le choix du capteur, plus particulièrement sa gamme spectrale, est dicté par les gammes spectrales des changements de couleurs provenant des réactifs.

Dans le contexte de l'invention, il faut comprendre par changement de couleur, l'évolution de la couleur d'un élément réactif lors de sa mise au contact avec un liquide biologique, par exemple et de manière non limitative, d'un changement de la couleur orange vers le bleu pour une indication d'acidité (ou encore dénoté 'pH') de liquide biologique, mais encore d'une apparition de couleur, c'est-à-dire de l'aspect blanc d'un l'élément réactif à une coloration rose lors de la détection de nitrites dans un liquide biologique au contact de l'élément réactif adéquat.

Le lecteur 14 comporte également un élément support 38 de cartouche s'étendant selon l'axe longitudinal L et comportant des moyens de fixations 40 lui permettant d'être fixé dans le boîtier 16, de préférence de manière solidaire avec la carte électronique 28 à circuit imprimé. Plus particulièrement, l'élément support 38 de cartouche comporte un fond de support 42 de cartouche s'étendant longitudinalement, et agencé en vis à vis et distant de la face supérieure de la carte électronique 28 à circuit imprimé. Selon ce premier mode de réalisation, le fond de support 42 de cartouche comporte une pluralité d'ouvertures 44, 46 de fond de support 42, chaque ensemble 30 émetteur-récepteur optique étant agencé en vis-à-vis d'une ouverture 44 du fond de support 42 de cartouche. De façon particulière, chaque source de lumière 34 d'un ensemble 30 émetteur-récepteur optique est séparée du capteur optique 36 du même ensemble 30 émetteur-récepteur optique par une paroi verticale permettant de supprimer les réflexions spéculaires.

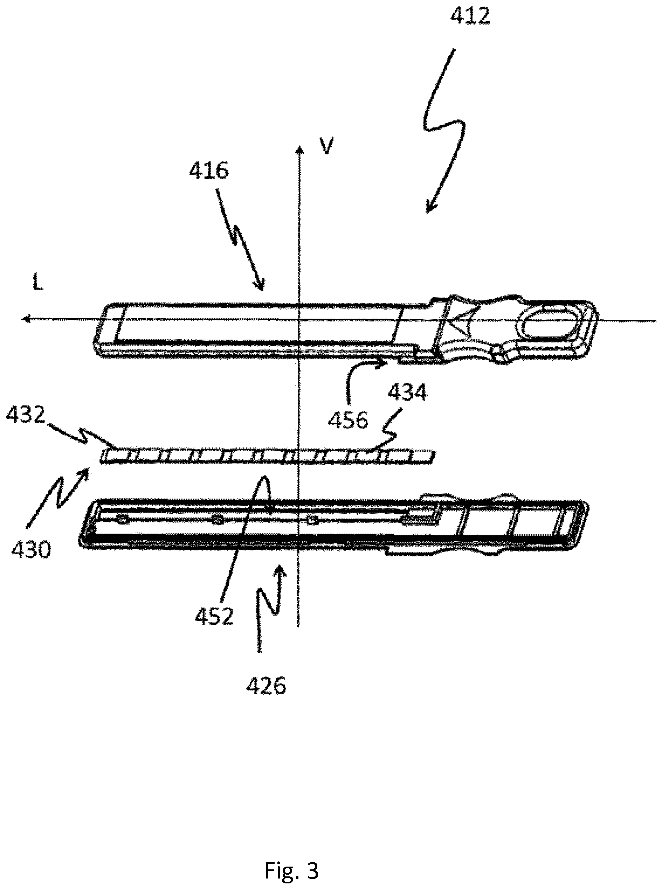

À cet effet, selon la figure 2 et la figure 3, et selon le premier mode de réalisation du lecteur 14, le système 10 d'analyse d'échantillon liquide biologique comprend la cartouche 412 de prélèvement d'échantillon liquide biologique de la figure 1, la cartouche 412 de prélèvement étant configurée pour être agencée en appui sur le fond de support 42 de cartouche du lecteur 14. La cartouche 412 de prélèvement comporte un boîtier 416 de cartouche s'étendant longitudinalement et comportant un fond de boîtier 426 de cartouche. La cartouche 412 de prélèvement comporte une pluralité d'éléments réactifs 432, 434 à changement de couleur agencés les uns à la suite des autres selon l'axe longitudinal L, chaque élément réactif 432, 434 étant agencé en fond de boîtier 426 de cartouche et en vis-à-vis d'une ouverture 452 du fond de boîtier 426 de cartouche. De façon optionnelle, la pluralité d'éléments réactifs 432, 434 peut être agencée sur une bandelette 430 de préférence autocollante et transparente ou translucide.

En présence de la cartouche 12 de prélèvement, l'unité électronique de contrôle 32 est configurée pour contrôler une commande récurrente d'un mode d'émission de lumière et de réception de la lumière réfléchie de chaque ensemble 30 émetteur-récepteur optique. L'unité électronique de contrôle 32 est configurée pour analyser un échantillon de liquide biologique par détection d'un changement de couleur de chaque élément réactif 432, 434 suite à une lecture optique de la couleur de chaque élément réactif 432, 434.

Selon la figure 2, afin de garantir une lecture optique fiable par le lecteur 14 du changement de couleur des éléments réactifs 432, 434 de la cartouche de prélèvement, le système 10 comprend un capteur de position 54 agencé dans le boîtier 16 de lecteur 14. Le capteur de position 54 est relié électriquement à l'unité de contrôle 32. Le capteur de position 54 est configuré pour détecter que chaque élément réactif 432, 434 de la cartouche est agencé en vis à vis d'un ensemble 30 émetteur-récepteur optique du lecteur 14. Lorsque le capteur de position 54 constate que chaque élément réactif 432, 434 de la cartouche est agencé en vis à vis d'un ensemble 30 émetteur-récepteur optique du lecteur 14, le capteur de position 54 est configuré pour envoyer un signal de commande à l'unité électronique 32 de contrôle autorisant l'unité de contrôle à effectuer une lecture optique de chaque élément réactif 432, 434 de manière récurrente.

Plus particulièrement, selon la figure 2, le capteur de position 54 comporte un interrupteur agencé sur la carte électronique 28 à circuit imprimé, et configuré pour coopérer avec une butée de position 456 de la cartouche 412 de prélèvement. En d'autres termes, la cartouche 412 de prélèvement comporte une butée de position 456 configurée pour heurter et déclencher un interrupteur agencé dans le lecteur 14 lorsque la cartouche 412 de prélèvement est correctement positionnée dans le lecteur 14, c'est-à-dire lorsque chaque élément réactif 432, 434 agencé dans la cartouche 412 de prélèvement est agencé en vis-à-vis d'un ensemble 30 émetteur-récepteur optique au travers de la pluralité d'ouvertures 44, 46 de fond de support 42 de cartouche du lecteur 14 et de l'ouverture 452 du fond de boîtier 426 de cartouche.

De façon particulière, et selon la figure 3, la butée de position 456 comprend un ergot agencé protubérant sur un rebord latéral du boîtier 416 de cartouche. La position selon l'axe longitudinal L de la butée de position 456 sur le rebord latéral du boîtier 416 de cartouche et la position selon l'axe longitudinal L de l'interrupteur doivent être calibrées de sorte que lorsque la butée de position 456 heurte l'interrupteur, chaque élément réactif 432, 434 de la cartouche soit agencé en vis à vis d'un ensemble 30 émetteur-récepteur optique du lecteur 14. La butée de position 456 sur le rebord latéral du boîtier 416 de cartouche, ou ergot, est de préférence agencée à proximité de l'extrémité droite de la cartouche de prélèvement de sorte à ne pas apporter de contraintes sur la longueur selon l'axe longitudinal L, de la cartouche 412 de prélèvement.

Il est à noter, à titre d'exemples non limitatifs, que de manière alternative, le capteur de position 54 peut comprendre une barrière infrarouge agencée de façon transversale sur l'élément support de cartouche 38, de préférence à l'extrémité de l'élément support de cartouche 38 opposée à l'ouverture latérale d'accès 22 du boîtier 16 de lecteur 14, de sorte que la barrière infrarouge puisse être coupée par l'extrémité gauche du boîtier 416 de cartouche lors de son insertion dans le lecteur 14. Alternativement, le capteur de position 54 peut comprendre un interrupteur type bouton poussoir agencé également à l'extrémité de l'élément support de cartouche 38, cet interrupteur pouvant être activé par écrasement de l'extrémité gauche du boîtier 416 de cartouche sur le bouton poussoir lors de son insertion dans le lecteur 14.

Selon la figure 2, afin de faciliter l'insertion de la cartouche 412 de prélèvement dans le lecteur 14, l'élément support de cartouche 38 comporte deux parois latérales 48, 50 agencées de manière opposée de part et d'autre du fond de support 42 de cartouche, chaque paroi latérale 48, 50 s'étendant longitudinalement depuis l'ouverture latérale d'accès 22. L'agencement des parois latérales 48, 50 de part et d'autre du fond de support 42 de cartouche forme un guide d'insertion 52 de cartouche de prélèvement.

Selon la figure 2, le lecteur 14 est configuré pour servir de support à la cartouche 412 de prélèvement lors du dépôt de liquide biologique permettant d'imbiber les éléments réactifs 432, 434. A cet effet, il convient que la cartouche 412 de prélèvement comprend une portion de dépôt 437 de liquide biologique accessible depuis l'extérieur du lecteur 14 lorsque la cartouche 412 de prélèvement est insérée dans le lecteur 14. Selon ce mode de réalisation, le lecteur 14 comporte un joint 58 d'étanchéité agencé dans l'ouverture latérale d'accès 22 du lecteur 14 de sorte à garantir une étanchéité de l'ouverture latérale d'accès 22 à tout liquide biologique pouvant être déposé sur la cartouche 412 de prélèvement après son insertion dans le lecteur 14. Le joint d'étanchéité 58 est configuré pour venir en compression autour de la portion de la cartouche 412 de prélèvement agencée au travers de l'ouverture latérale d'accès 22 lorsque la cartouche 412 de prélèvement est correctement positionnée dans le lecteur 14.

Afin de faciliter la prise en main du lecteur, notamment durant le dépôt de liquide biologique sur la portion de dépôt 437 de liquide de la cartouche 412 de prélèvement insérée dans le lecteur 14, le fond de boîtier 26 de lecteur 14 comprend sur sa face inférieure un moyen de préhension 60 manuel du lecteur 14, le moyen de préhension 60 comportant de manière particulière au moins une surface formant un creux bombé vers l'intérieur du boîtier 16 de lecteur 14 et s'étendant longitudinalement suivant une forme de type arc de cercle.

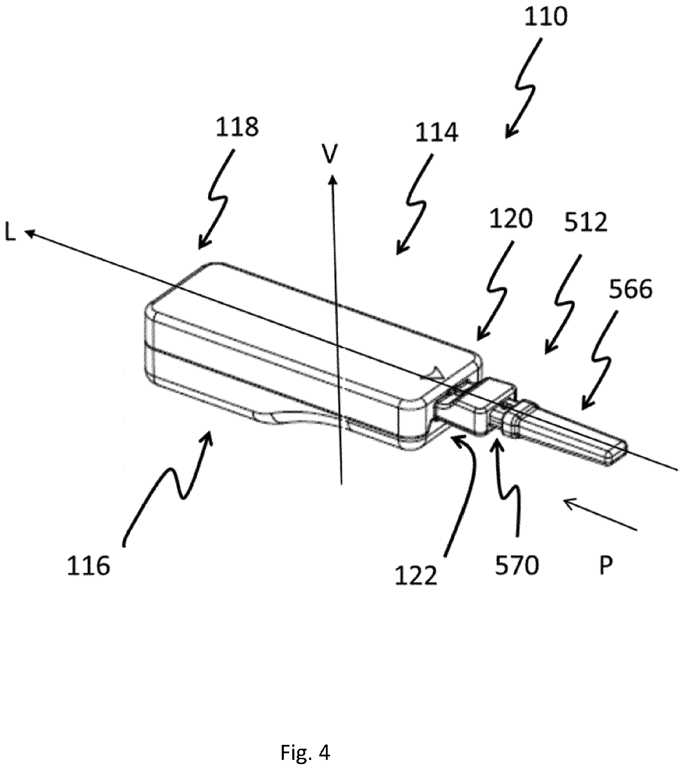

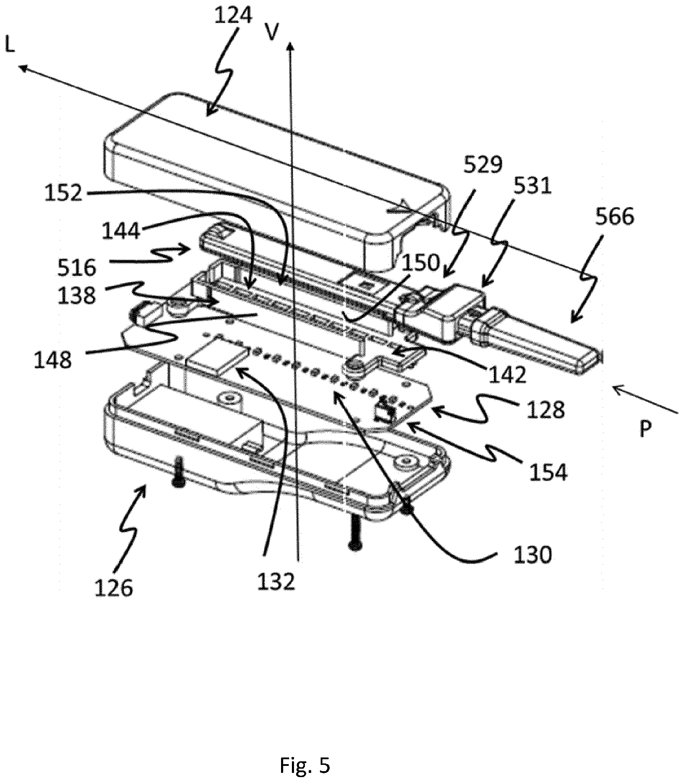

Selon la figure 4, la figure 5 et la figure 6, un second mode de réalisation d'un système 110 d'analyse d'échantillon liquide biologique ne relevant pas de l'invention est représenté. Le second mode de réalisation, de façon similaire au premier mode de réalisation comporte une cartouche 512 de prélèvement d'échantillon liquide biologique destinée à être insérée dans un lecteur 114 portable d'analyse de liquide biologique. Le lecteur 114 comprend un boîtier 116 de lecteur 114 s'étendant globalement selon l'axe longitudinal L entre deux extrémités 118, 120 latérales gauche et droite. Le boîtier 116 de lecteur 114 comprend une ouverture latérale d'accès 122 destinée à une insertion ou une extraction de la cartouche 512 de prélèvement selon l'axe longitudinal L. La cartouche 512 de prélèvement est configurée pour être agencée sur un fond de support 142 de cartouche d'un élément support 138 de cartouche du lecteur 114.

De manière similaire au premier mode de réalisation, l'élément support 138 de cartouche comporte deux parois latérales 148, 150 agencées de manière opposée de part et d'autre du fond de support 142 de cartouche, chaque paroi latérale 148, 150 s'étendant longitudinalement depuis l'ouverture latérale d'accès 122. L'agencement des parois latérales 148, 150 de part et d'autre du fond de support 142 de cartouche forme alors un guide d'insertion 152 de cartouche de prélèvement.

De manière similaire au premier mode de réalisation, le lecteur 114 comporte également une carte électronique 128 à circuit imprimé agencée en fond de boîtier 126 de lecteur 114. La carte électronique 128 à circuit imprimé comporte une pluralité d'ensembles 130 émetteur-récepteur optique, les ensembles 30 émetteur-récepteur optique de la pluralité d'ensembles 30 étant agencés les uns à la suite des autres selon l'axe longitudinal L. Le lecteur 114 comporte une unité électronique de contrôle 132, avantageusement agencée sur la carte électronique 128 à circuit imprimé, et reliée électriquement à la pluralité d'ensembles 130 émetteur-récepteur optique. Le lecteur 114 comporte également un capteur de position 154 avantageusement agencé sur la carte électronique 128 à circuit imprimé.

Également de manière similaire au premier mode de réalisation, le fond de support 142 de cartouche comporte une pluralité d'ouvertures 144, 146 de fond de support 142, chaque ensemble 130 émetteur-récepteur optique étant agencé en vis-à-vis d'une ouverture 144 du fond de support 142 de cartouche.

Le second mode de réalisation diffère notamment du premier mode de réalisation en ce que le lecteur 114 et la cartouche 512 de prélèvement sont configurés pour coopérer ensemble de sorte à rendre fiable la diffusion d'un échantillon de liquide biologique depuis une portion de dépôt 537 de la cartouche 512 de prélèvement jusqu'aux éléments réactifs 532, 534 de la cartouche 512 de prélèvement.

A cet effet, le système 110 comporte une poignée 566 configurée pour être agencée solidaire avec la cartouche 512 de prélèvement et permettant d'exercer une poussée P sur la cartouche 512 selon l'axe longitudinal L lors de l'insertion de la cartouche 512 de prélèvement en translation dans le lecteur 114 au travers de l'ouverture latérale d'accès 122 du lecteur 114.

Selon le second mode de réalisation, la poignée 566 est agencée à l'extérieure du boîtier 116 de lecteur 114 de sorte à recouvrir au moins partiellement une portion d'une bande absorbante réservoir 536 de la cartouche 512 de prélèvement s'étendant partiellement à l'extérieur de la cartouche 512 de prélèvement. La bande absorbante réservoir 536 est configurée pour comprendre la portion de dépôt 537 de liquide biologique agencée à l'extérieur de la cartouche 512 de prélèvement et configurée pour être recouverte par la poignée 566. La poignée 566 agit donc comme un capuchon par-dessus la portion de dépôt 537 de liquide biologique de la bande absorbante réservoir 536.

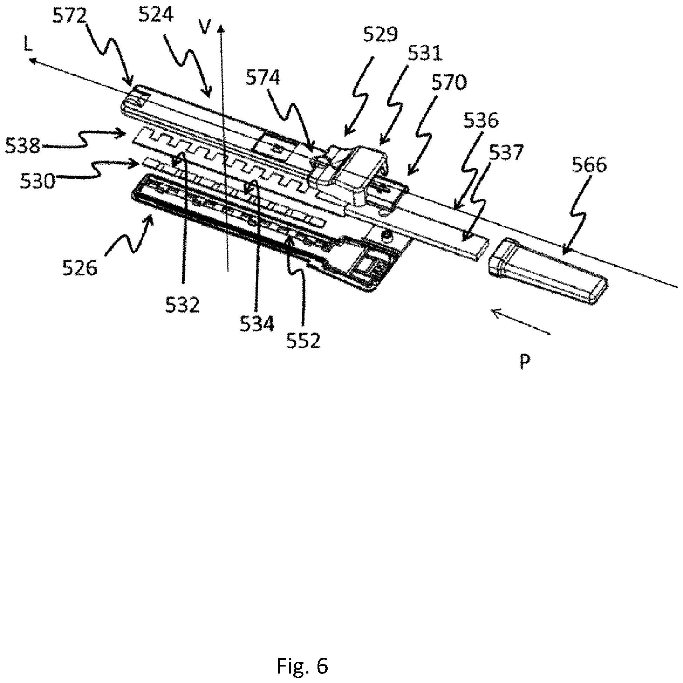

Selon la figure 6, la bande absorbante réservoir 536 est agencée dans un support mobile 570 configuré pour coulisser dans une portion de coulissement 531 du boîtier 516 de cartouche, la portion de coulissement 531 étant agencée à l'extrémité droite de la cartouche 512 de prélèvement. La poignée 566, agencée autour de la portion de dépôt 537 de la bande absorbante réservoir 536 est en liaison mécanique avec le support mobile 570, la poignée 566 étant configurée pour exercer une poussée P en translation sur la bande absorbante réservoir 536.

Selon le second mode de réalisation, la cartouche 512 de prélèvement comporte une bande de diffusion 538 de liquide biologique s'étendant longitudinalement et agencée dans le boîtier 516 de cartouche par-dessus la pluralité d'éléments réactifs 532, 534. La bande de diffusion 538 est configurée pour être en contact direct avec chaque élément réactif 532, 534 de la cartouche 512 de prélèvement. Avantageusement, la pluralité d'éléments réactifs 532, 534 est agencée sur la face supérieure d'une bandelette 530 agencée dans le fond de boîtier 526 de cartouche, chaque élément réactif 532, 534 étant agencé en vis-à-vis d'une ouverture 552 du fond de boîtier 526 de cartouche par transparence au travers de la bandelette 530.

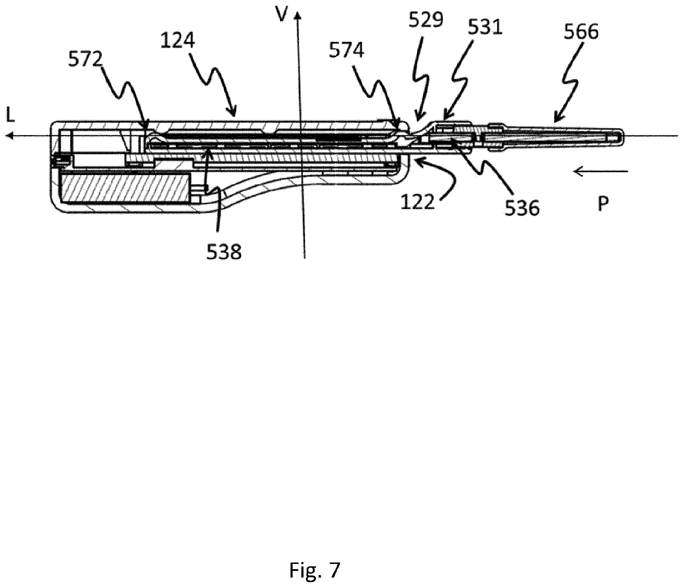

Selon la figure 6 et la figure 7, et selon le second mode de réalisation, avant une insertion maximale de la cartouche 512 de prélèvement dans le lecteur 114, c'est-à-dire lorsque la cartouche 512 de prélèvement n'est pas encore correctement positionnée dans le lecteur 114, ou encore lorsque les éléments réactifs 532, 534 de la cartouche 412 de prélèvement ne sont pas tous agencés en vis-à-vis d'un ensemble 130 émetteur-récepteur optique de lecteur 114, la bande absorbante réservoir 536 n'est pas en contact avec la bande de diffusion 538.

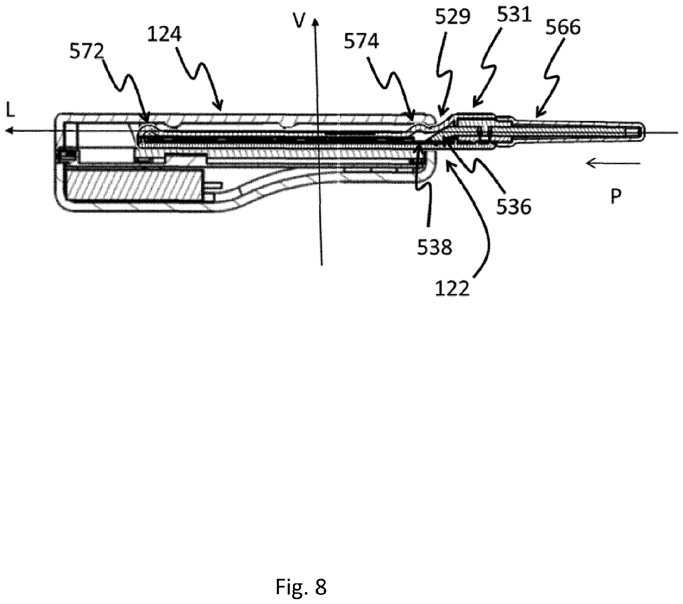

Selon la figure 8, suite à l'insertion en butée de la cartouche 512 de prélèvement dans le lecteur 114, une poussée P selon l'axe longitudinal L de la poignée 566 a pour effet n'ont plus de faire avancer selon l'axe longitudinal L la cartouche 512 de prélèvement en insertion dans le lecteur 114, mais a pour effet de pousser la bande absorbante réservoir 536 au contact direct de la bande de diffusion 538. Plus particulièrement, la bande absorbante réservoir 536 est configurée pour être poussée par la poignée 566 selon un mouvement de translation et guidée par le support mobile 570 contre la surface interne d'une portion de transition 529 du boîtier 516 de la cartouche 512 de prélèvement, la surface interne de la portion de transition 529 ayant pour effet de faire plier la bande absorbante réservoir 536 vers la bande de diffusion 538 de sorte à ce que le contact se fasse entre les deux bandes 536, 538.

Il est à noter que l'insertion en butée de la cartouche 512 de prélèvement est effective soit lorsque la portion de transition 529 vient en butée contre le pourtour de l'ouverture latérale d'accès 122 du lecteur 114, ou alternativement lorsque l'extrémité gauche de la cartouche 512 de prélèvement, c'est-à-dire l'extrémité opposée à la poignée 566 vient en butée dans le boîtier 116 de lecteur 114 lors de son insertion maximale dans le lecteur 114. La solution d'une butée obtenue par la coopération entre la portion de transition 529 de la cartouche 512 de prélèvement et le pourtour de l'ouverture latérale d'accès 122 du lecteur 114 a l'avantage de ne pas contraindre la conception du lecteur 114 en fonction de la longueur, selon l'axe longitudinal L, de la cartouche 512 de prélèvement.

Selon la figure 6, la figure 7 et la figure 8, afin de fiabiliser la mise en contact entre la bande de diffusion 538 de la cartouche 512 de prélèvement et les éléments réactifs 532, 534, le boîtier 516 de la cartouche 512 de prélèvement comporte deux bossages 572, 574 agencés sur la paroi supérieure du boîtier 516 de la cartouche 512 de prélèvement, ou encore couvercle 524 de la cartouche 512 de prélèvement et faisant saillie vers l'extérieur du boîtier 516 de la cartouche 512. Le couvercle 524 de la cartouche 512 de prélèvement est agencé par-dessus la bande de diffusion 538. Selon la figure 7 et la figure 8, lors de l'insertion de la cartouche 512 de prélèvement dans le lecteur 114, les deux bossages 572, 574 viennent en frottement avec la face interne du couvercle 124 du lecteur 114 de sorte à exercer une pression verticale vers le bas sur la bande de diffusion 538. La bande de diffusion 538 ainsi pressée est maintenue au contact des éléments réactifs 532, 534 de la cartouche de prélèvement.

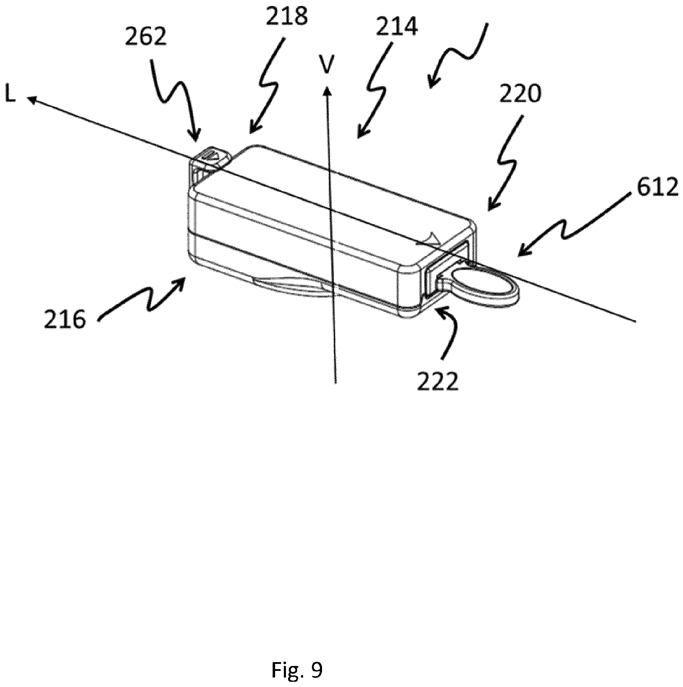

Selon la figure 9, la figure 10 et la figure 11, un troisième mode de réalisation d'un système 210 d'analyse d'échantillon liquide biologique est représenté. Le troisième mode de réalisation, de façon similaire au premier mode de réalisation et au second mode de réalisation, comporte une cartouche 612 de prélèvement d'échantillon liquide biologique destinée à être insérée dans un lecteur 214 portable d'analyse de liquide biologique. Le lecteur 214 comprend un boîtier 216 de lecteur 214 s'étendant globalement selon l'axe longitudinal L entre deux extrémités 218, 220 latérales gauche et droite. Le boîtier 216 de lecteur 214 comprend une ouverture latérale d'accès 222 destinée à une insertion ou une extraction de la cartouche 612 de prélèvement selon l'axe longitudinal L.

De manière similaire au premier mode de réalisation, le lecteur 214 comporte également une carte électronique 228 à circuit imprimé agencée en fond de boîtier 226 de lecteur 214. La carte électronique 228 à circuit imprimé comporte une pluralité d'ensembles 230 émetteur-récepteur optique, les ensembles 230 émetteur-récepteur optique de la pluralité d'ensembles 230 étant agencés les uns à la suite des autres selon l'axe longitudinal L. Le lecteur 214 comporte une unité électronique de contrôle 232, avantageusement agencée sur la carte électronique 228 à circuit imprimé, et reliée électriquement à la pluralité d'ensembles 230 émetteur-récepteur optique. Le lecteur 214 comporte également un capteur de position 254 avantageusement agencé sur la carte électronique 228 à circuit imprimé.

De manière similaire au premier et second mode de réalisation, le lecteur 214 comporte un élément support de cartouche 238 comportant deux parois latérales 248, 250 agencées de manière opposée de part et d'autre du fond de support 242 de cartouche, chaque paroi latérale 248, 250 s'étendant longitudinalement depuis l'ouverture latérale d'accès 222. L'agencement des parois latérales 248, 250 de part et d'autre du fond de support 242 de cartouche forme alors un guide d'insertion 252 de cartouche de prélèvement.

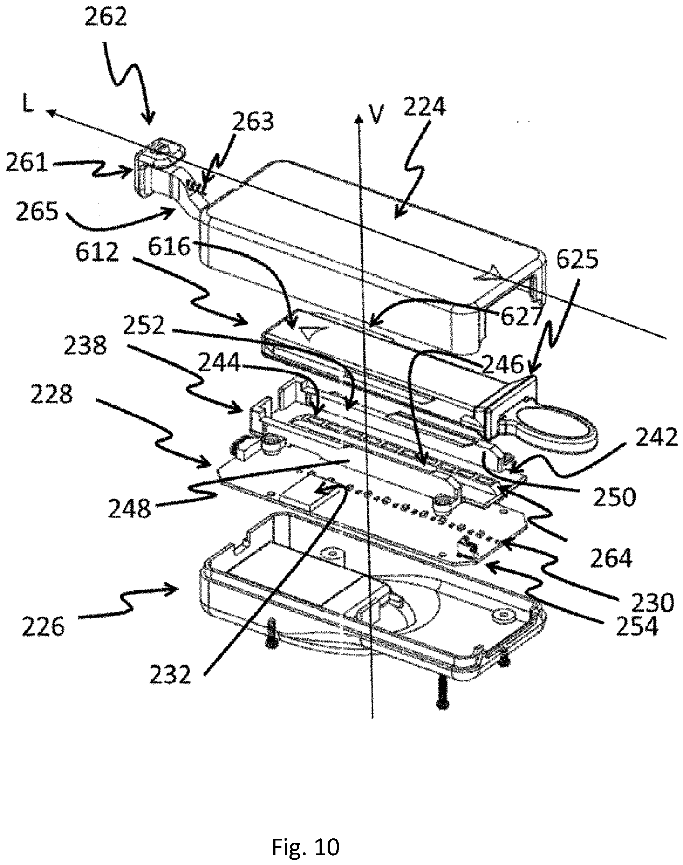

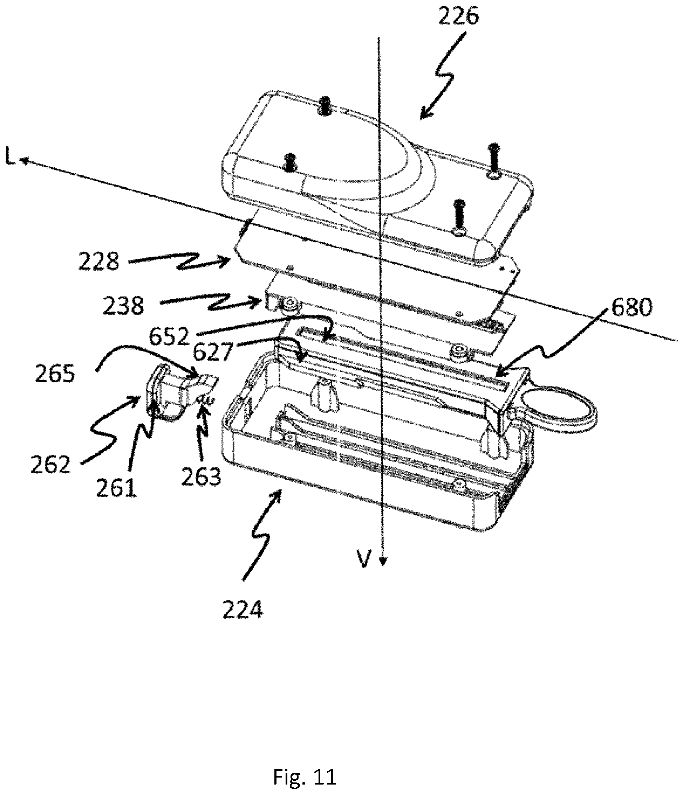

De manière particulière, tel qu'illustré aux figures 10 et 11, le boîtier 616 de cartouche peut comprendre des ailettes 627 latérales de guidage configurées pour pouvoir coulisser dans des rainures agencées sur les parois latérales 248, 250 du guide d'insertion 252 de cartouche de prélèvement de sorte à garantir une insertion de la cartouche 612 de prélèvement dans le lecteur 214 selon un mouvement de translation.

Selon la figure 10 et la figure 11, le troisième mode de réalisation diffère du premier et du second mode de réalisation en ce que le fond de support 242 de cartouche de l'élément support 238 de cartouche du lecteur 214 comporte une portion protubérante 264 s'étendant longitudinalement sur laquelle est agencée une pluralité d'ouvertures 244, 246 de fond de support 242.

Selon le troisième mode de réalisation, et de manière similaire au premier et au second mode de réalisation, la cartouche 612 de prélèvement comporte une pluralité d'éléments réactifs 632, 634 à changement de couleur agencés les uns à la suite des autres selon l'axe longitudinal L, chaque élément réactif 632, 634 étant agencé en fond de boîtier 626 de cartouche et en vis-à-vis d'une ouverture 652 du fond de boîtier 626 de cartouche. Pour les besoins du troisième mode de réalisation, le matériau du pourtour 680 de l'ouverture 652 de fond de boîtier 626 de cartouche 612 de prélèvement est un matériau souple tel qu'un plastique tendre.

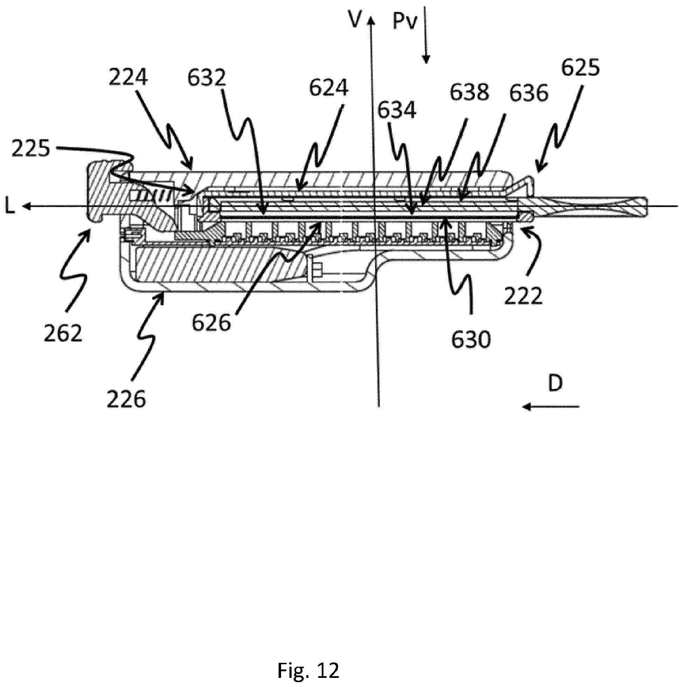

Selon la figure 12, et selon le troisième mode de réalisation, la cartouche 612 de prélèvement comporte une bande de diffusion 638 de liquide biologique, s'étendant longitudinalement, agencée dans le boîtier 616 de cartouche par-dessus la pluralité d'éléments réactifs 632, 634.Lorsque la cartouche 612 de prélèvement n'est pas encore correctement positionnée dans le lecteur 214, la bande de diffusion 638 de liquide biologique n'est pas en contact avec chaque élément réactif 632, 634.

Selon la figure 12, le système 210 comporte deux éléments de poussée configurés pour pousser la cartouche 612 de prélèvement selon une poussée verticale Pv en direction du fond de support 242 de cartouche. Plus particulièrement, un premier élément de poussée 625 comporte une première portion pentue oblique de type biseau agencée sur le couvercle 624 de cartouche. La première portion pentue oblique s'étend de manière montante depuis la surface du couvercle 624 de cartouche vers l'extrémité droite de la cartouche 612 de prélèvement. Un second élément de poussée 225 comporte une seconde portion pentue oblique de type biseau agencée sur la face interne du couvercle 224 de lecteur 214 et dirigé pentue vers le bas selon la direction D d'insertion de la cartouche 612 de prélèvement.

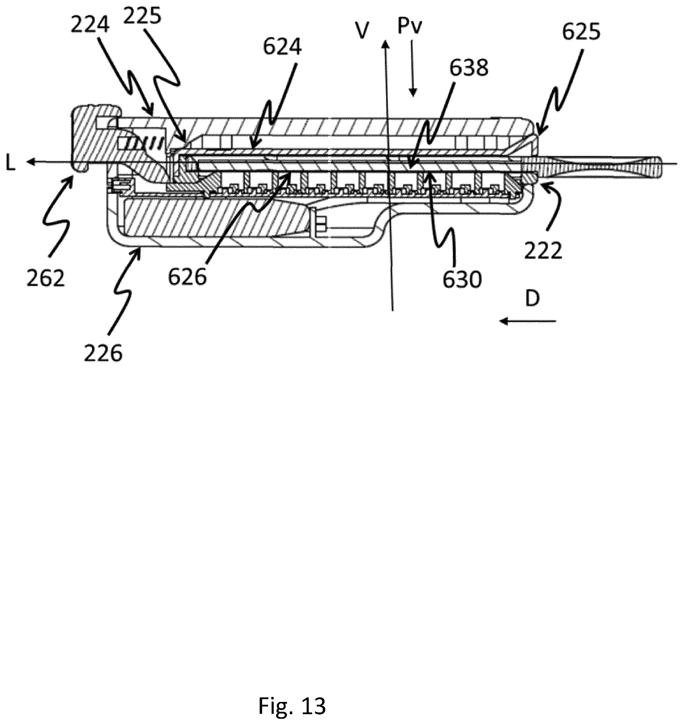

À cet effet, selon la figure 13, lors de l'insertion de la cartouche 612 de prélèvement dans le lecteur 214, le premier élément de poussée 625 vient au contact de l'ouverture latérale d'accès 222 de lecteur. Le second élément de poussée 225 vient au contact de l'extrémité gauche de la cartouche 612 de prélèvement. Lorsque la cartouche 612 de prélèvement est poussée vers sa position d'insertion maximale, elle est poussée en direction du fond de support 242 de cartouche de sorte que le pourtour 680 de l'ouverture 652 de fond de boîtier 626 de cartouche 612 de prélèvement en matériau souple vient s'écraser contre la portion protubérante 264 du fond de support 242 de cartouche. À cet effet, chaque élément réactif 632, 634 vient au contact de la bande de diffusion 638. De manière avantageuse, selon ce troisième mode de réalisation, la bande de diffusion 638 peut faire également office de bande absorbante réservoir 636, de sorte que la bande de diffusion 638 et la bande absorbante réservoir 636 consiste en un seul et unique élément absorbant, par exemple et de manière non limitative, de type papier buvard absorbant.

Selon la figure 9, la figure 10 et la figure 11, le lecteur 214 comporte un bouton d'éjection 262 configuré pour éjecter la cartouche 612 de prélèvement. Le bouton d'éjection 262 est agencé sur la paroi latérale de l'extrémité gauche du boîtier 216 de lecteur 214, c'est-à-dire sur la paroi du lecteur 214 opposée à l'ouverture latérale d'accès 222. Le bouton d'éjection 262 est agencé au travers de la paroi latérale de l'extrémité gauche du boîtier 216, le bouton d'éjection 262 comportant une portion de manipulation 261 manuelle agencée à l'extérieur du boîtier 216 de lecteur 214. Le bouton d'éjection 262 comporte également une portion d'éjection 265 s'étendant depuis la portion de manipulation 261 jusqu'à l'intérieur de boitier 216 de lecteur 214. La portion d'éjection 265 est configurée pour venir coulisser en translation selon l'axe longitudinal L par-dessus le fond de support 242 de cartouche de sorte à pouvoir heurter et éjecter par l'ouverture latérale d'accès 222 une cartouche 612 de prélèvement ayant été agencée dans le lecteur 214. A cet effet et de manière optionnelle, le bouton d'éjection 262 comporte un moyen ressort 263 venant en appui dans le boîtier 216 de lecteur 214 et permettant un mouvement de poussée avec retour en position initial du bouton d'éjection 262. L'avantage du bouton d'éjection 262 est de pouvoir extraire une cartouche 612 de prélèvement sans avoir à la manipuler.

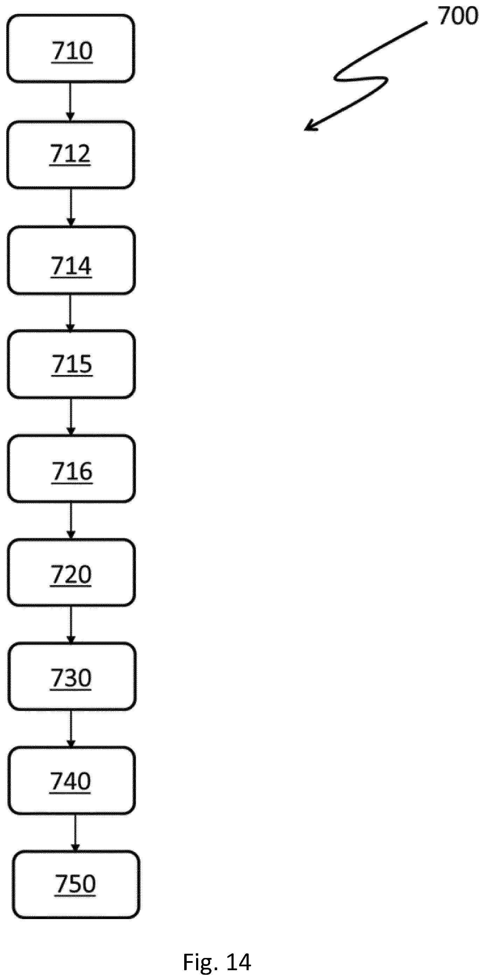

Selon la figure 14, un exemple de méthode 700 d'analyse d'échantillon de liquide biologique comporte une pluralité d'étapes. La méthode présentée, s'applique pairticulièrement aux systèmes d'analyse d'échantillon de liquide biologique des figures précédentes. La méthode comporte une première étape d'insertion 710 de la cartouche 412, 512, 612 de prélèvement dans le lecteur 14, 114, 214. Particulièrement, l'insertion se fait par une ouverture latérale d'accès 22, 122, 222 du lecteur 14, 114, 214, l'ouverture latérale d'accès 22, 122, 222 permettant l'insertion selon une direction D longitudinale ou l'extraction de la cartouche 412, 512, 612 de prélèvement. Une étape suivant l'insertion de la cartouche de prélèvement 412, 512, 612 consiste en une lecture optique 720 récurrente de la pluralité d'éléments réactifs 432, 532, 632 de la cartouche 412, 512, 612. On entend par lecture optique, dans le cadre de l'invention, une lecture par réflexion de lumière sur chaque élément réactif 432, 532, 632 au travers des ouvertures 452, 552, 652 de fond de boîtier 426, 526, 626 de cartouche et de la bandelette 430, 530, 630, permettant la détection par le lecteur 14, 114, 214 d'un changement de couleur des éléments réactifs 432, 532, 632 et donc permettant l'analyse automatique par le lecteur 14, 114, 214 des propriétés de l'échantillon du liquide biologique à analyser.

Faisant suite à l'étape de lecture optique 720 de manière récurrente, la méthode comprend une étape de détection 730 d'un premier changement de couleur d'au moins un élément réactif 432, 532, 632 de la pluralité d'éléments réactifs 432, 532, 632, afin de pouvoir effectuer une étape de datation 740 du premier changement de couleur détecté et également une étape d'analyse 750 de l'échantillon de liquide biologique de la cartouche 412, 512, 612 suite à la détection 130 d'un changement de couleur d'au moins un élément réactif 432, 532, 632 de la cartouche.

À titre d'exemple non limitatif, un changement de couleur d'un élément réactif 432, 532, 632 peut être détecté par un calcul récurrent effectué par l'unité électronique de contrôle 32, 132, 232 d'un ratio de chaque quantité de lumière de chaque couleur primaire détectée. Le lecteur 14, 114, 214 peut alors également comprendre une mémoire électronique dans la quelle peut être mémorisé des ratios de couleurs primaires associés à chaque élément réactif 432, 532, 632 de sorte à pouvoir déterminer la présence d'un composé du liquide biologique présent sur chaque élément réactif 432, 532, 632.

De manière optionnelle, pour un système 10, 110, 210 selon lequel un capteur de position 54, 154, 254 est agencé dans le boîtier 16, 116, 216 de lecteur 14, 114, 214, la méthode peut comprendre, avant l'étape de lecture optique 720, une étape de détection 712 par un capteur de position 54, 154, 254 de l'agencement de chaque élément réactif 432, 532, 632 de la cartouche 412, 512, 612 en vis-à-vis d'un ensemble émetteur-récepteur 30, 130, 230 optique du lecteur 14, 114, 214, de sorte à également comprendre une étape supplémentaire d'autorisation 714 de l'étape de lecture optique 720 récurrente sous condition de la détection 712 de l'agencement de chaque élément réactif 432, 532, 632 de la cartouche 412, 512, 612 en vis-à-vis d'un ensemble 30, 130, 230 émetteur-récepteur optique du lecteur 14, 114, 214.

De manière optionnelle, pour un système 110, 210 selon lequel une bande de diffusion 538, 638 de liquide biologique est agencée par-dessus la pluralité d'éléments réactifs 532, 632, la méthode 700 peut comporter une étape de mise en contact 716 direct de la bande diffusion 538, 638 sur la pluralité d'éléments réactifs suite à l'étape d'insertion de la cartouche 512, 612 dans le lecteur 114, 214 par écrasement de la cartouche 512, 612 de prélèvement par le lecteur 114, 214.

De manière optionnelle, pour un système 110 selon lequel la cartouche 512 de prélèvement comporte une bande absorbante réservoir 536 configurée pour le dépôt de liquide biologique, la méthode 700 peut comporter une étape de mise en contact direct 715 d'une portion de la bande réservoir absorbante 536 sur la bande de diffusion 538 par poussée P longitudinale de la cartouche 512 de prélèvement lors de son insertion dans le lecteur 114.

Il est à noter que l'invention est particulièrement avantageuse pour l'analyse de liquide biologique de type urine. Les éléments réactifs 432, 532, 632 à changement de couleur au contact de l'urine agencés dans une cartouche de prélèvement, selon l'invention, peuvent être analysés par un lecteur 14, 114, 214 d'analyse selon l'invention, de sorte que le lecteur puisse par exemple et de manière non limitative, donner une information relative à la surveillance de l'acidité de l'urine via son potentiel hydrogène, noté pH, ou encore la densité urinaire et sa concentration en créatinine. La concentration en acide urique est également un facteur de surveillance possible.

Il est à noter que pour avoir un accès convivial à l'analyse du lecteur 14, 114, 214, il est avantageux que le lecteur 14, 114, 214 comporte une interface de communication avec ou sans fil permettant de communiquer avec tout objet portable servant d'interface de lecture pour un utilisateur de l'invention. À titre d'exemples non limitatifs, on citera comme objet portable, un téléphone portable de type smartphone, ou encore une tablette informatique ou un ordinateur.

Il doit être bien entendu que la description détaillée de l'objet de l'Invention, donnée uniquement à titre d'illustration, ne constitue en aucune manière une limitation, les équivalents techniques étant également compris dans le champ de la présente invention.

## Revendications

1. Système (210) d'analyse d'échantillon de liquide biologique comprenant :
- un lecteur (214) portable d'analyse d'échantillon de liquide biologique comportant :
o un boîtier (216) de lecteur (214) s'étendant selon un axe longitudinal (L) entre deux extrémités latérales, le boîtier (216) de lecteur (214) comprenant :
▪ une ouverture latérale d'accès (222) destinée à une insertion ou une extraction de la cartouche (612) de prélèvement selon l'axe longitudinale (L);
▪ une partie inférieure comprenant un fond de boîtier (226) de lecteur (214);
▪ un couvercle (224) de lecteur (214) destiné à la fermeture du boîtier (216) de lecteur (214);
∘ une carte électronique (228) à circuit imprimé agencée en fond de boîtier (226) de lecteur (214);
∘ une pluralité d'ensembles (230) émetteur-récepteur optique agencée sur la face supérieure de la carte électronique (228) à circuit imprimé, les ensembles (230) émetteur-récepteur optique de la pluralité d'ensembles (230) étant agencés les uns à la suite des autres selon l'axe longitudinal (L) ;
o un élément support (238) de cartouche s'étendant longitudinalement comportant :
▪ un fond de support (242) de cartouche s'étendant longitudinalement, et agencé en vis à vis et distant de la face supérieure de la carte électronique (228) à circuit imprimé; chaque ensemble (230) émetteur-récepteur optique étant agencé en vis-à-vis d'une ouverture (244) du fond de support (242) de cartouche;
o une unité électronique de contrôle (232) reliée électriquement à la pluralité d'ensembles (230) émetteur-récepteur optique; l'unité électronique de contrôle (232) étant configurée pour commander chaque ensemble (230) émetteur-récepteur optique dans un mode d'émission de lumière et de réception de la lumière réfléchie de sorte à pouvoir effectuer une lecture optique à des fins d'analyse de liquide biologique.
- une cartouche (612) de prélèvement d'échantillon liquide biologique configurée pour être agencée en appui sur le fond de support (242) de cartouche du lecteur (214), la cartouche (612) comportant :
o un boîtier (616) de cartouche s'étendant longitudinalement entre deux extrémités latérales, et comprenant un fond de boîtier (626) de cartouche;
o une pluralité d'éléments réactifs (632) à changement de couleur agencés les uns à la suite des autres selon l'axe longitudinal (L); chaque élément réactif (632) étant agencé en fond de boîtier (626) de cartouche et en vis-à-vis d'une ouverture (652) du fond de boîtier (626) de cartouche;
o une bande de diffusion (638) de liquide biologique, s'étendant longitudinalement, agencée dans le boîtier (616) de cartouche par-dessus la pluralité d'éléments réactifs (632, 634); la bande de diffusion (638) étant configurée pour être en contact direct avec chaque élément réactif (632) de la pluralité d'éléments réactifs (632);
o une portion souple (680) du fond (626) de boîtier de cartouche agencée en regard de chaque élément réactif (632, 634) de la pluralité d'éléments réactifs (632, 634);
o un couvercle (624) de cartouche agencé par-dessus la bande de diffusion (638);
- l'unité électronique de contrôle (232) configurée pour analyser un échantillon de liquide biologique par détection d'un changement de couleur de chaque élément réactif (632) suite à une lecture optique
- le système (210) comportant au moins un élément de poussée (225) coopérant avec le couvercle (624) de cartouche et configuré pour :
o pousser la cartouche (612) en direction du fond de support (242) de cartouche ;
o écraser la portion souple (680) du fond de boîtier (626) de cartouche contre une portion protubérante (264) du fond de support (242) de cartouche lors de l'insertion de la cartouche (612) dans le lecteur (214) de sorte que chaque élément réactif (632, 634) soit en contact avec la bande de diffusion (638).

2. Système (210) selon la revendication 1 **caractérisé en ce qu'**il comprend :
- un capteur de position (254) agencé dans le boîtier (216) de lecteur et relié électriquement à l'unité de contrôle (232), le capteur de position (254) étant configuré pour :
o détecter que chaque élément réactif (632) de la cartouche ( 612) est agencé en vis à vis d'un ensemble (30, 130, 230) émetteur-récepteur optique du lecteur (214);
o envoyer un signal de commande, à l'unité électronique (232) de contrôle, de lecture optique de chaque élément réactif (632) de manière récurrente suite à la détection de l'agencement en vis-à-vis de chaque élément réactif (632) avec un ensemble (230) émetteur récepteur optique.

3. Système (210) selon l'une quelconque des revendication 1 ou 2 **caractérisé en ce que** l'élément support (238) de cartouche comporte deux parois latérales s'étendant longitudinalement depuis l'ouverture latérale d'accès (222) et agencées de part et d'autre du fond de support (242) formant un guide (252) d'insertion de cartouche de prélèvement.

4. Méthode (700) d'analyse d'échantillon de liquide biologique, la méthode (700) étant mise en œuvre par le système (210) d'analyse d'échantillon de liquide biologique de l'une quelconque des revendications 1 à 3 ; la cartouche (612) comportant une bande de diffusion (638) de liquide biologique agencée par-dessus la pluralité d'éléments réactifs (632) ; la méthode (700) comportant des étapes de :
- insertion (710) de la cartouche (612) de prélèvement dans le lecteur (214);
- mise en contact (716) direct de la bande diffusion (638) sur la pluralité d'éléments réactifs suite à l'étape d'insertion de la cartouche (612) dans le lecteur (214) par écrasement de la cartouche (612) par le lecteur (214) ;
- lecture optique (720) récurrente de la pluralité d'éléments réactifs (632) de la cartouche (612) faisant suite à l'insertion (110) de la cartouche (612);
- détection (730) d'un premier changement de couleur d'au moins un élément réactif (632) de la pluralité d'éléments réactifs (632) suite à l'étape de lecture optique (120) récurrente ;
- datation (740) du premier changement de couleur détecté;
- analyse (750) de l'échantillon de liquide biologique de la cartouche (612) suite à la détection (130) d'un changement de couleur d'au moins un élément réactif (632) de la cartouche (612).

5. Méthode (700) selon la revendication 4, le lecteur (214) du système (210) comportant un capteur de position (254) agencé dans le boîtier (216) de lecteur (214), la méthode (700) comprenant des étapes préalables à l'étape de lecture optique (720) récurrente, de :
- détection (712) par le capteur de position (254) de l'agencement de chaque élément réactif (632) de la cartouche (612) en vis-à-vis d'un ensemble émetteur-récepteur (230) optique du lecteur (214);
- autorisation (714) de l'étape de lecture optique (720) récurrente sous condition de la détection (712) de l'agencement de chaque élément réactif (632) de la cartouche (612) en vis-à-vis d'un ensemble (230) émetteur-récepteur optique du lecteur (214).

6. Méthode (700) selon l'une quelconque des revendications 4 ou 5, la cartouche (512) comportant une bande absorbante réservoir (536) configurée pour le dépôt de liquide biologique, la méthode (700) comportant une étape de mise en contact direct (715) d'une portion de la bande réservoir absorbante (536) sur la bande de diffusion (538) par poussée (P) longitudinale de la cartouche (512) de prélèvement lors de son insertion dans le lecteur (114).

## Patentansprüche

1. System (210) zur Analyse einer biologischen Flüssigkeitsprobe, umfassend:
- ein tragbares Lesegerät (214) zur Analyse einer biologischen Flüssigkeitsprobe, das Folgendes aufweist:
o ein Gehäuse (216) des Lesegeräts (214), das sich entlang einer Längsachse (L) zwischen zwei seitlichen Enden erstreckt, wobei das Gehäuse (216) des Lesegeräts (214) Folgendes umfasst:
▪ eine seitliche Zugangsöffnung (222) zum Einführen oder Herausziehen der Entnahmekartusche (612) entlang der Längsachse (L);
▪ einen unteren Teil, der einen Gehäuseboden (226) des Lesegeräts (214) umfasst;
▪ einen Deckel (224) des Lesegeräts (214) zum Verschließen des Gehäuses (216) eines Lesegeräts (214);
o eine Leiterplatten-Elektronikkarte (228), die an dem Gehäuseboden (226) des Lesegeräts (214) angeordnet ist;
o eine Vielzahl von optischen Sender-Empfänger-Baugruppen (230), die auf der oberen Seite der Leiterplatte-Elektronikkarte (228) angeordnet sind, wobei die optischen Sender-Empfänger-Baugruppen (230) der Vielzahl von Baugruppen (230) nacheinander entlang der Längsachse (L) angeordnet sind;
o ein sich längs erstreckendes Kartuschenträgerelement (238), das Folgendes aufweist:
▪ einen sich längs erstreckenden Kartuschenträgerboden (242), der gegenüberliegend und von der oberen Seite der Leiterplatte (228) entfernt angeordnet ist; wobei jede optische Sender-Empfänger-Baugruppe (230) gegenüber einer Öffnung (244) des Kartuschenträgerbodens (242) angeordnet ist;
o eine elektronische Steuereinheit (232), die elektrisch mit der Vielzahl von optischen Sender-Empfänger-Baugruppen (230) verbunden ist, wobei die elektronische Steuereinheit (232) so eingerichtet ist, dass sie jede optische Sender-Empfänger-Baugruppe (230) in einem Modus zum Senden von Licht und zum Empfangen von reflektiertem Licht steuert, so dass eine optische Ablesung für die Analyse biologischer Flüssigkeiten durchgeführt werden kann.
- eine Kartusche (612) zur Entnahme einer biologischen Flüssigkeitsprobe, die so eingerichtet ist, dass sie auf dem Trägerboden (242) der Kartusche des Lesegeräts (214) aufliegt, wobei die Kartusche (612) Folgendes aufweist:
o ein Kartuschengehäuse (616), das sich längs zwischen zwei seitlichen Enden erstreckt und einen Kartuschengehäuseboden (626) umfasst;
o eine Vielzahl von farbändernden Reaktionselementen (632), die nacheinander entlang der Längsachse (L) angeordnet sind; wobei jedes Reaktionselement (632) an dem Kartuschengehäuseboden (626) und gegenüber einer Öffnung (652) des Kartuschengehäusebodens (626) angeordnet ist;
o ein sich längs erstreckendes Diffusionsband (638) einer biologischen Flüssigkeit, das in dem Kartuschengehäuse (616) über der Vielzahl von Reaktionselementen (632, 634) angeordnet ist; wobei das Diffusionsband (638) so eingerichtet ist, dass es in direktem Kontakt mit jedem Reaktionselement (632) der Vielzahl von Reaktionselementen (632) steht;
o einen weichen Abschnitt (680) des Kartuschengehäusebodens (626), der gegenüber jedem Reaktionselement (632, 634) der Vielzahl von Reaktionselementen (632, 634) angeordnet ist;
o einen Kartuschendeckel (624), der über dem Diffusionsband (638) angeordnet ist;
- die elektronische Steuereinheit (232), die so eingerichtet ist, dass sie eine biologische Flüssigkeitsprobe durch Erkennung einer Farbänderung jedes Reaktionselements (632) infolge einer optischen Ablesung analysiert
- wobei das System (210) mindestens ein Druckelement (225) aufweist, das mit dem Kartuschendeckel (624) zusammenwirkt und für Folgendes eingerichtet ist:
o Drücken der Kartusche (612) in Richtung des Kartuschenträgerbodens (242);
o Zerdrücken des weichen Teils (680) des Kartuschengehäusebodens (626) gegen einen hervorstehenden Teil (264) des Kartuschenträgerbodens (242), wenn die Kartusche (612) in das Lesegerät (214) eingeführt wird, so dass jedes Reaktionselement (632, 634) Kontakt mit dem Diffusionsband (638) hat.

2. System (210) nach Anspruch 1, **dadurch gekennzeichnet, dass** es Folgendes umfasst:
- einen Positionssensor (254), der in dem Gehäuse (216) eines Lesegeräts angeordnet und elektrisch mit der Steuereinheit (232) verbunden ist, wobei der Positionssensor (254) für Folgendes eingerichtet ist:
o Erkennen, dass jedes Reaktionselement (632) der Kartusche (612) gegenüber einer optischen Sender-Empfänger-Baugruppe (30, 130, 230) des Lesegeräts (214) angeordnet ist;
o Senden eines Steuersignals an die elektronische Steuereinheit (232) zur wiederholten optischen Ablesung jedes Reaktionselements (632) nach der Erkennung der gegenüberliegenden Anordnung jedes Reaktionselements (632) mit einer optischen Sender-Empfänger-Baugruppe (230).

3. System (210) nach einem der Ansprüche 1 oder 2, **dadurch gekennzeichnet, dass** das Kartuschenträgerelement (238) zwei Seitenwände aufweist, die sich längs von der seitlichen Zugangsöffnung (222) erstrecken und auf beiden Seiten des Trägerbodens (242) angeordnet sind, der eine Führung (252) zu dem Einführen einer Entnahmekartusche bildet.

4. Verfahren (700) zur Analyse einer biologischen Flüssigkeitsprobe, wobei das Verfahren (700) durch das System (210) zur Analyse einer biologischen Flüssigkeitsprobe nach einem der Ansprüche 1 bis 3 implementiert ist; die Kartusche (612) ein Diffusionsband (638) einer biologischen Flüssigkeit aufweist, das auf der Vielzahl von Reaktionselementen (632) angeordnet ist; wobei das Verfahren (700) folgende Schritte aufweist:
- Einführen (710) der Entnahmekartusche (612) in das Lesegerät (214);
- direktes Inkontaktbringen (716) des Diffusionsbands (638) mit der Vielzahl von Reaktionselementen infolge des Schritts des Einführens der Kartusche (612) in das Lesegerät (214) durch Zerdrücken der Kartusche (612) durch das Lesegerät (214);
- wiederkehrendes optisches Ablesen (720) der Vielzahl von Reaktionselementen (632) der Kartusche (612), die infolge des Einführens (110) der Kartusche (612) erfolgt;
- Erkennen (730) einer ersten Farbänderung von mindestens einem Reaktionselement (632) der Vielzahl von Reaktionselementen (632) infolge des Schritts der wiederkehrenden optischen Ablesung (120);
- Datieren (740) der ersten erkannten Farbänderung;
- Analysieren (750) der biologischen Flüssigkeitsprobe der Kartusche (612) nach dem Erkennen (130) einer Farbänderung von mindestens einem Reaktionselement (632) der Kartusche (612).

5. Verfahren (700) nach Anspruch 4, wobei das Lesegerät (214) des Systems (210) einen Positionssensor (254) aufweist, der in dem Gehäuse (216) des Lesegeräts (214) angeordnet ist, wobei das Verfahren (700) folgende Schritte vor dem Schritt der wiederkehrenden optischen Ablesung (720) umfasst:
- Erkennen (712) durch den Positionssensor (254) der Anordnung jedes Reaktionselements (632) der Kartusche (612) gegenüber einer optischen Sender-Empfänger-Baugruppe (230) des Lesegeräts (214);
- Autorisieren (714) des Schritts der wiederkehrenden optischen Ablesung (720) unter der Bedingung, dass die Anordnung jedes Reaktionselements (632) der Kartusche (612) gegenüber einer optischen Sender-Empfänger-Baugruppe (230) des Lesegeräts (214) erkannt (712) wird.

6. Verfahren (700) nach einem der Ansprüche 4 oder 5, wobei die Kartusche (512) ein absorbierendes Tankband (536) aufweist, das für die Ablagerung biologischer Flüssigkeit eingerichtet ist, wobei das Verfahren (700) einen Schritt des direkten Inkontaktbringens (715) eines Abschnitts des absorbierenden Tankbands (536) durch längsseitiges Drücken (P) der Entnahmekartusche (512) beim Einführen in das Lesegerät (114) in direkten Kontakt mit dem Diffusionsband (538) gebracht wird.

## Claims

1. System (210) for analysing a biological liquid sample comprising:
- a portable reader (214) for analysing a biological liquid sample comprising:
o a housing (216) of the reader (214) extending along a longitudinal axis (L) between two lateral ends, the housing (216) of the reader (214) comprising:
▪ a lateral access opening (222) intended for an insertion or an extraction of the collection cartridge (612) along the longitudinal axis (L);
▪ a lower portion comprising a housing bottom (226) of the reader (214);
▪ a cover (224) of the reader (214) intended for closing the housing (216) of the reader (214) ;
∘ an electronic card (228) with printed circuit arranged in the housing bottom (226) of the reader (214);
∘ a plurality of optical transmitter-receiver assemblies (230) arranged on the upper face of the electronic card (228) with printed circuit, the optical transmitter-receiver assemblies (230) of the plurality of assemblies (230) being arranged one after the other along the longitudinal axis (L);
o a cartridge support element (238) extending longitudinally comprising:
▪ a cartridge support bottom (242) which is extending longitudinally, and arranged facing and distant from the upper face of the electronic card (228) with printed circuit; each optical transmitter-receiver assembly (230) being arranged facing an opening (244) of the cartridge support bottom (242);
o an electronic monitoring unit (232) electrically connected to the plurality of optical transmitter-receiver assemblies (230); the electronic monitoring unit (232) being configured to control each optical transmitter-receiver assembly (230) in a mode of emitting light and receiving the reflected light so as to be able to perform an optical reading for the purpose of analysing a biological liquid;
- a cartridge (612) for collecting a biological liquid sample configured to be arranged to bear on the cartridge support bottom (242) of the reader (214), the cartridge (612) comprising:
o a cartridge housing (616) extending longitudinally between two lateral ends, and comprising a cartridge housing bottom (626);
o a plurality of colour-changing reactive elements (632) arranged one after the other along the longitudinal axis (L); each reactive element (632) being arranged in the cartridge housing bottom (626) and facing an opening (652) of the cartridge housing bottom (626);
o a biological liquid diffusion band (638), extending longitudinally, arranged in the cartridge housing (616) over the top of the plurality of reactive elements (632, 634); the diffusion band (638) being configured to be in direct contact with each reactive element (632) of the plurality of reactive elements (632);
o a flexible portion (680) of the cartridge housing bottom (626) arranged opposite each reactive element (632, 634) of the plurality of reactive elements (632, 634);
o a cartridge cover (624) arranged over the top of the diffusion band (638);
- the electronic monitoring unit (232) configured to analyse a biological liquid sample by detecting a colour change of each reactive element (632) following an optical reading
- the system (210) comprising at least one pushing element (225) cooperating with the cartridge cover (624) and configured to:
o push the cartridge (612) in the direction of the cartridge support bottom (242);
o crush the flexible portion (680) of the cartridge housing bottom (626) against a protruding portion (264) of the cartridge support bottom (242) when inserting the cartridge (612) into the reader (214) such that each reactive element (632, 634) is in contact with the diffusion band (638).

2. System (210) according to claim 1 **characterised in that** it comprises:
- a position sensor (254) arranged in the reader housing (216) and electrically connected to the monitoring unit (232), the position sensor (254) being configured to:
o detect that each reactive element (632) of the cartridge (612) is arranged facing an optical transmitter-receiver assembly (30, 130, 230) of the reader (214);
o send a control signal, to the electronic monitoring unit (232), for optical reading of each reactive element (632) in a recurrent manner following the detection of the arrangement facing each reactive element (632) with an optical transmitter-receiver assembly (230).

3. System (210) according to any one of claims 1 or 2, **characterised in that** the cartridge support element (238) comprises two lateral walls extending longitudinally from the lateral access opening (222) and arranged on either side of the support bottom (242) forming a guide (252) for inserting the collection cartridge.

4. Method (700) for analysing a biological liquid sample, the method (700) being implemented by the system (210) for analysing a biological liquid sample of any one of claims 1 to 3; the cartridge (612) comprising a biological liquid diffusion band (638) arranged over the top of the plurality of reactive elements (632); the method (700) comprising steps of:
- inserting (710) the collection cartridge (612) into the reader (214);
- direct contacting (716) of the diffusion band (638) on the plurality of reactive elements following the step of inserting the cartridge (612) into the reader (214) by crushing the cartridge (612) by the reader (214) ;
- recurrent optical reading (720) of the plurality of reactive elements (632) of the cartridge (612) following the insertion (110) of the cartridge (612);
- detecting (730) a first colour change of at least one reactive element (632) of the plurality of reactive elements (632) following the recurrent optical reading step (120);
- dating (740) the first detected colour change;
- analysing (750) the biological liquid sample of the cartridge (612) following the detection (130) of a colour change of at least one reactive element (632) of the cartridge (612).

5. Method (700) according to claim 4, the reader (214) of the system (210) comprising a position sensor (254) arranged in the housing (216) of the reader (214), the method (700) comprising steps prior to the recurrent optical reading step (720), of:
- detecting (712) by the position sensor (254) of the arrangement of each reactive element (632) of the cartridge (612) facing an optical transmitter-receiver assembly (230) of the reader (214);
- authorising (714) the recurrent optical reading step (720) on condition of the detection (712) of the arrangement of each reactive element (632) of the cartridge (612) facing an optical transmitter-receiver assembly (230) of the reader (214).

6. Method (700) according to any one of claims 4 or 5, the cartridge (512) comprising an absorbent reservoir band (536) configured for the deposition of biological liquid, the method (700) comprising a step of direct contacting (715) of a portion of the absorbent reservoir band (536) on the diffusion band (538) by longitudinal pushing (P) of the collection cartridge (512) when it is inserted into the reader (114).
